(19) Europäisches Patentamt
European Patent Office
Office européen des brevets

(11) Veröffentlichungsnummer: 0 207 397
A2

(12) # EUROPÄISCHE PATENTANMELDUNG

(21) Anmeldenummer: 86108430.9

(22) Anmeldetag: 20.06.86

(51) Int.Cl.[4]: A 61 K 31/44
//(A61K31/44, 31:365)

(30) Priorität: 02.07.85 DE 3523540

(43) Veröffentlichungstag der Anmeldung: 07.01.87 Patentblatt 87/2

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Gross, Rainer, Dr.
Platzhoffstrasse 23
D-5600 Wuppertal 1(DE)

(72) Erfinder: Schramm, Matthias, Dr.
Paffratherstrasse 38
D-5000 Köln 80(DE)

(54) **Dihydropyridin Kombinationspräparat.**

(57) Die vorliegende Erfindung betrifft ein festes Kombinationspräparat der allgemeinen Formel I

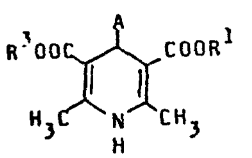

(I)

in welcher $R^1$, $R^3$ und A die in der Beschreibung angegebene Bedeutung haben enthaltend ein Dihydropyridin und Isosorbid-5-mononitrat, welches zur Langzeitbehandlung von Herzerkrankungen, insbesondere von Erkrankungen der Coronarien und des Herzmuskels eingesetzt werden kann.

EP 0 207 397 A2

Croydon Printing Company Ltd

0207397

Bayer Aktiengesellschaft
Konzernverwaltung RP
Patentabteilung

5090 Leverkusen, Bayerwerk
KS/Gh

## Dihydropyridin Kombinationspräparat

Die vorliegende Erfindung betrifft ein festes Kombinationspräparat enthaltend ein Dihydropyridin und ein Nitrat, welches zur Langzeitbehandlung von Herzerkrankungen, insbesondere von Erkrankungen der Coronarien und des Herzmuskels eingesetzt werden kann.

Die verwendeten Dihydropyridine sind bekannt (Britisches Patent Nr. 1 358 951). Auch ihre Verwendung als Herz- und Kreislaufmittel ist bekannt.

Die verwendeten Nitrate (insbesondere Isosorbid-5-mononitrat (IS-5-MN) und Isosorbiddinitrat (ISDN)) sind bekannt. Sie wirken ebenfalls gefäßdilatierend, wobei sich bei der Behandlung von Herzerkrankungen eine bevorzugte Wirkung an den Venen zeigt. Bei der Langzeitbehandlung von Herzerkrankungen mit solchen Nitropräparaten zeigt sich häufig eine unerwünschte Toleranzentwicklung, die eine Steigerung der Dosierung erforderlich macht bzw. keine ausreichende therapeutische Wirkung mehr zeigt.

Die Erfindung betrifft feste, oral applizierbare Arzneizubereitungen zur Verwendung bei der langzeitigen Bekämpfung von Herzerkrankungen, insbesondere Erkrankungen der Coronarien und des Herzmuskels enthaltend 1 bis 40 mg eines Dihydropyridins der allgemeinen Formel I

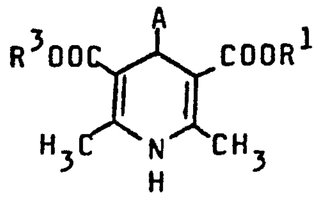

(I)

in welcher

$R^1$ und $R^3$ immer voneinander verschieden sind und für Alkyl mit 1 bis 12 Kohlenstoffatomen stehen, welches gegebenenfalls durch Fluor oder Chlor ein- oder mehrfach substituiert ist oder welches gegebenenfalls durch ein Sauerstoffatom in der Kette unterbrochen ist, und

A für den Rest 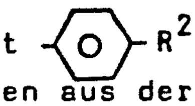, wobei $R^2$ für einen oder zwei Substituenten aus der Gruppe Nitro, Chlor, Trifluormethyl steht, oder für 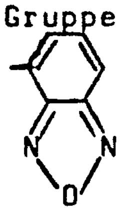 steht

und 0,1 bis 100 mg Nitrate sowie gegebenenfalls übliche Hilfs- und Trägerstoffe.

Von besonderem Interesse sind solche Kombinationspräparate, welche 5 bis 30 mg des Dihydropyridins und 10 bis 30 mg Isosorbid-5-mononitrat oder 5 bis 50 mg Isosorbiddinitrat enthalten.

Besonders bevorzugte Dihydropyridine sind Nisoldipin und Nitrendipin.

Überraschenderweise wird bei der Anwendung der erfindungsgemäßen Kombination die Toleranzentwicklung gegenüber der Nitratkomponente verhindert. Dies läßt sich in Versuchen an isolierten Geweben nachweisen. So läßt sich beispielsweise die Toleranzentwicklung von Nitraten durch Bestimmung ihrer Wirkung auf die Serotonin-induzierte Kontraktion von Pulmonalvenen nachweisen (U. Borchard et al., Med. Klin. Sondernummer 1 (1985), 21-23).

Setzt man in diesen Versuchen den Substanzeffekt direkt nach Substanzgabe als 100 %, so ist die zeitliche Abnahme dieses Effektes ein Maß für die Toleranzentwicklung. Nach fünf Stunden (bzw. nach 2 bei den ISDN-Versuchen) ergeben sich die folgenden Werte:

| Konzentration (g/ml) | | | | Wirkungsverlust |
|---|---|---|---|---|
| ISMN | ISDN | Nitrendipin | Nisoldipin | nach 5 (2) Std. |
| $10^{-4}$ | 0 | 0 | 0 | 42 |
| $3 \times 10^{-4}$ | 0 | 0 | 0 | 48 |
| 0 | $3 \times 10^{-6}$ | 0 | 0 | 100 |
| 0 | $10^{-5}$ | 0 | 0 | 100 |
| 0 | 0 | $10^{-6}$ | 0 | 2 |
| 0 | 0 | $3 \times 10^{-6}$ | 0 | 0 |
| 0 | 0 | 0 | $10^{-7}$ | 0 |
| 0 | 0 | 0 | $3 \times 10^{-7}$ | 0 |
| $10^{-4}$ | 0 | $10^{-6}$ | 0 | 9 |
| 0 | $10^{-6}$ | $10^{-6}$ | 0 | 4 |
| $10^{-4}$ | 0 | 0 | $10^{-7}$ | 8 |
| 0 | $10^{-6}$ | 0 | $10^{-7}$ | 8 |

Dieser unerwartete Befund ist insbesondere bei der notwendigen Langzeittherapie der genannten Herzerkrankungen von entscheidendem Vorteil.

Darüberhinaus versetzt die vorliegende Erfindung den Fachmann in die Lage, den gleichen therapeutischen Effekt mit wesentlich reduzierter Dosierung der Wirkstoffe zu erreichen. Hierdurch werden unerwünschte Nebenwirkungen reduziert. Dies ist ebenfalls bei der Langzeittherapie von großem Vorteil.

Die nachfolgenden Beispiele erläutern spezielle Ausführungsformen der erfindungsgemäßen Kombinationspräparate, ohne beschränkende Wirkung zu haben.

## Herstellungsbeispiele

### Beispiel 1

10 g Nisoldipin (mikrofein) werden mit 80 g einer Verreibung von IS-5-MN in Lactose (25 %ig), 41,4 g Maisstärke, 15 g Lactose und 20 g Avicel gemischt und dann mit einer Lösung aus 0,8 g Natriumlaurylsulfat in 12 g Polyvinylpyrrolidon (PVP 25) granuliert. Das Granulat wird getrocknet, gesiebt und mit 0,8 g Magnesiumstearat gemischt. Aus dieser Mischung werden Tabletten mit einem mittleren Tablettengewicht von 180 mg gepreßt oder die Mischung wird in Steckkapseln mit 180 mg Kapselinhalt abgefüllt.

0207397

## Patentansprüche

1. Feste oral applizierbare Arzneizubereitungen enthaltend 1 bis 40 mg eines Dihydropyridins der allgemeinen Formel I

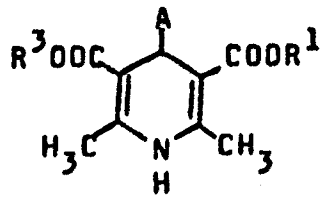

(I)

in welcher

$R^1$ und $R^3$ immer voneinander verschieden sind und für Alkyl mit 1 bis 12 Kohlenstoffatomen stehen, welches gegebenenfalls durch Fluor oder Chlor ein- oder mehrfach substituiert ist oder welches gegebenenfalls durch ein Sauerstoffatom in der Kette unterbrochen ist, und

A für den Rest 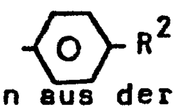, wobei $R^2$ für einen oder zwei Substituenten aus der Gruppe Nitro, Chlor, Trifluormethyl steht, oder für 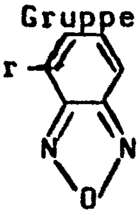 steht

und 0,1 bis 100 mg Nitrate, sowie gegebenenfalls übliche Hilfs- und Trägerstoffe.

2. Arzneizubereitungen gemäß Anspruch 1, dadurch gekennzeichnet, daß sie 5 bis 30 mg des Dihydropyridins enthalten.

3. Arzneizubereitungen gemäß Anspruch 1, dadurch gekennzeichnet, daß sie als Dihydropyridin Nisoldipin oder Nitrendipin enthalten.

4. Arzneizubereitungen gemäß Anspruch 1, dadurch gekennzeichnet, daß sie 10 bis 30 mg Isosorbid-5-mononitrat oder 5 bis 50 mg Isosorbiddinitrat enthalten.

5. Verfahren zur Herstellung von Arzneizubereitungen gemäß Ansprüchen 1 bis 3, dadurch gekennzeichnet, daß man ein Dihydropyridin der allgemeinen Formel I gemäß Anspruch 1 mit Nitraten, gegebenenfalls unter Verwendung von üblichen Hilfs- und Trägerstoffen in eine geeignete Applikationsform überführt.

6. Verwendung von Arzneizubereitungen gemäß Ansprüchen 1 bis 3 bei der Bekämpfung von Herzerkrankungen.

7. Verwendung von Dihydropyridinen der allgemeinen Formel I gemäß Anspruch 1 und Nitraten bei der Herstellung von Arzneimitteln zur Bekämpfung von Herzerkrankungen.